(19) Europäisches Patentamt
European Patent Office
Office européen des brevets

(11) EP 3 940 085 A1

(12) EUROPEAN PATENT APPLICATION
published in accordance with Art. 153(4) EPC

(43) Date of publication:
**19.01.2022 Bulletin 2022/03**

(21) Application number: **20732643.0**

(22) Date of filing: **13.03.2020**

(51) International Patent Classification (IPC):
***C12Q 1/6883*** *(2018.01)*

(52) Cooperative Patent Classification (CPC):
**C12Q 1/6883;** C12Q 2600/156; C12Q 2600/158

(86) International application number:
**PCT/ES2020/070180**

(87) International publication number:
**WO 2020/188133 (24.09.2020 Gazette 2020/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **15.03.2019 ES 201930240**

(71) Applicant: **Universidad Del Pais Vasco Euskal Herriko Unibertsitatea**
**48940 Leioa (Vizcaya) (ES)**

(72) Inventors:
- **BILBAO CATALA, José Ramón**
  **48940 Leioa, Vizcaya (ES)**
- **FERNÁNDEZ JIMÉNEZ, Nora**
  **48940 Leioa, Vizcaya (ES)**

(74) Representative: **ABG Intellectual Property Law, S.L.**
**Avenida de Burgos, 16D**
**Edificio Euromor**
**28036 Madrid (ES)**

# (54) METHOD FOR DIAGNOSING CELIAC DISEASE BASED ON THE LEVEL OF EXPRESSION OF THE UBE2L3 GENE

(57) The present invention relates to methods for the diagnosis of celiac disease based on the quantification of the expression level of the UBE2L3 gene, as well as of the relative expression of isoform 2 of the UBE2L3 gene with respect to the total expression of the said gene. Likewise, the invention relates to kits with the means needed to perform the diagnosis according to the methods of the invention.

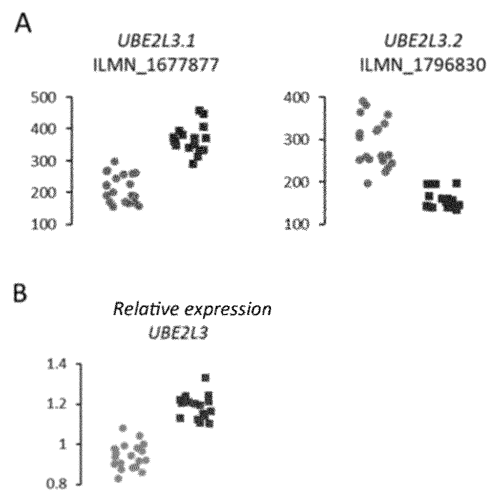


FIG. 2

EP 3 940 085 A1

SEQUENCE LISTING

<110> UNIVERSIDAD DEL PAÍS VASCO / EUSKAL HERRIKO UNIBERTSITATEA

<120> MÉTODO DE DIAGNÓSTICO DE ENFERMEDAD CELIACA BASADO EN EL NIVEL DE EXPRESIÓN DEL GEN UBE2L3

<130> P16820PC00

<150> P201930240
<151> 2019-03-15

<160> 7

<170> PatentIn version 3.5

<210> 1
<211> 225
<212> DNA
<213> H.sapiens

<400> 1
aaacgctgag gcccagtctg tgcggttcac ttgcgttcct ccacgcgccc cccagcacag 60

tgggaagcac acagtaggtg ctccgctctg ctcctgtgcc ccgccccgcg gcccctcccc 120

cgctccagga agtgcggggg ctccagccgc ccggccggcc gcgatgcatt ctggggaagg 180

agcagcacca aatccaagat ggcggccagc aggaggctga tgaag 225

<210> 2
<211> 96
<212> DNA
<213> H.sapiens

<400> 2
gagcttgaag aaatccgcaa atgtgggatg aaaaacttcc gtaacatcca ggttgatgaa 60

gctaatttat tgacttggca agggcttatt gttcct 96

<210> 3
<211> 187
<212> DNA
<213> H.sapiens

<400> 3
gacaaccctc catatgataa gggagccttc agaatcgaaa tcaactttcc agcagagtac 60

ccattcaaac caccgaagat cacatttaaa acaaagatct atcacccaaa catcgacgaa 120

aaggggcagg tctgtctgcc agtaattagt gccgaaaact ggaagccagc aaccaaaacc 180

gaccaag 187

<210> 4
<211> 2520
<212> DNA
<213> H.sapiens

<400> 4

```
taatccagtc cctcatagca ctggtgaatg acccccagcc tgagcacccg cttcgggctg      60
acctagctga agaatactct aaggaccgta aaaaattctg taagaatgct gaagagttta     120
caaagaaata tggggaaaag cgacctgtgg actaaaatct gccacgattg gttccagcaa     180
gtgtgagcag agaccccgtg cagtgcattc agacaccccg caaagcagga ctctgtggaa     240
attgacacgt gccaccgcct ggcgttcgct tgtggcagtt actaactttc tacagttttc     300
ttaatcaaaa gtggtctagg taacctgtaa agaaaggatt aaaaatttaa gatgttctag     360
ttctgctctc tttgttttaa aaatcactgc ttcaatctac ttcaaaagaa tggtgtttct     420
tttcttgtcc aattttatcc aaaatcttca agttacattt aacccataag gtttaaaaaa     480
aaggaaaaaa aacggttgtg gttccctttc ttccctaccc ttgccactcc cactttctgg     540
caccgagttt atttttcact tacttacttc cccagacccc gggctcgcct ccacaaagga     600
gaagagactg ccctggcggt cctggtggct tttcttagca tgtgtggcac tgttgcccag     660
tgtgggagtt ggtttaaatt ctcctgactc cagtttataa catcctttta aaaaatttaa     720
aaacaaacag ccacacccct cctccagtcc ttctcctcag ttcttgtgtg aaactccagc     780
tgatgttacc acagtaacat cagttaattg ggcaagccct gatgtcagtg tgtgtaactg     840
acctctggcc tggcctgcac agagaagccc tataatcaca ggtctgtggt ggccccgaaa     900
tggggggcct gctagtcagg aggatgctgt gcacactgtg tgtgatgaat ctcgccagaa     960
aggctcctga ggtcccaggt tggcacttct ccctgcagcc attgtagaag atctgctggt    1020
ccttgcaggc aaagctacag ccagaatgtc cgtttgaaac tcctagctca tctgtcaccg    1080
agcttcatcc gaatgtgcca cggagcttgc tctccacttc ctccgtgcag tggccctgcc    1140
acagccctcc ctcggcacac tttgaccctt tgtaggattg gaattagcag gactcggcta    1200
tttaaagcac cagtctgggg tcgcctgggc ccctgctgac cccctcctcc agagcagcca    1260
gcccagcccg ggaacaagac ggacttcctc tcccttcgga ctcacagcct ttgcagagtc    1320
aagctccact tgaagctcac tcagtaatat cctttcaatg tgttttatat tgttttgact    1380
gccttttttt gtagaaataa aaattgacct tagaatttat cgtcagataa acttgtaaag    1440
atttgaatat taatgtcttt tcaaggcaaa tgggattgtc cccgcactag tagagaatcc    1500
atgtcgctct gacaccccaa ggaagccgac gatccaaatg ccgtgtgtca ccaaccccgc    1560
ttctgccact ggcggcttcc cttcttggct cttggggggg actagatcct gtggagaaga    1620
tgacttaaac tttgcttttt gttttaattt taattctata acttgagatc tttccggggc    1680
ctacaggcgt gtaagacagc ttggtctggt ctgtgcagaa gtggggagtg atgggcaggt    1740
tcggcagcct aacattgttc aggcgcatgg cccctgcggt gtgtacacga actcggcttc    1800
ttttgtccta ggtacgccaa gggcaggttt ctggagactc ccttgtgccc gggatggcaa    1860
gggcaccggg ctggcgtttc cacatctgtc ttcattagca gaaaagtgat gatggatttt    1920
```

atttcactca cactccagtt tgtaataaaa tgccaaattc tgtcagctat ccaaacaagc 1980

caccatttgt tcttgttgct tctctggatc cagaaatgtt gccattcttg gaaactgtcc 2040

cattgcttcg tatttctgcc aacgtagctc tgcctgcctg tcaacccctc actgcactct 2100

gctcatcacg ggaggatacc tgtgtgccgg cagcccctca gggactctca gccctggcac 2160

tggcacccca gggttggccc cgtcagcaga ggcttggctt tcgagccagt gggtgtctct 2220

cctttgggcc tgggcggctt gctcctgcca gccatgcctt cagggtaggc tctgagcaag 2280

ctggcgaaca gccctggctg ctccaaaacc aaaaagctgg gtcctctgga ggaggggcga 2340

gctgtggagc agccacccac tgctgcccca agctcactca ggaattcaca cccgcctggt 2400

ttcttgaagt gtgctgggtc cttccctctg ctccctactc cccaccacgg cagagaatag 2460

gctttctaag atgctgcgat cccgttctgc tgcccgtaat aaaaatgctc tcagacactg 2520

<210> 5
<211> 109
<212> DNA
<213> H.sapiens

<400> 5
aaacttcagc gttcccaatt atggcttctc tcagatccag ccttgaagtt ctttgacctc 60

ctcaattcac aacctgtagc tgactttagc cacccacaag tacagaaaa 109

<210> 6
<211> 204
<212> DNA
<213> H.sapiens

<400> 6
agtatgcagg tcgctgcagg gacccgaggc gacacgcgcc tgcaggaggt ggcactcctg 60

ccgcagctct tcgatctgct tgtccttggc cagcggcgcg ctcgcctgct ccgacaggtc 120

ccgagcgcgc tggcgggcaa agacttggca cagctccagg ccggcgcaac tctcgccggg 180

tataggcgcg ctcacggccc ggag 204

<210> 7
<211> 648
<212> DNA
<213> H.sapiens

<400> 7
agcaccaaat ccaagatggc ggccagcagg aggctgatga aggtaaaagc cattctctgg 60

cagcggccgg gcgtggggcg gcgtcctagg ctccggatcc ccgaggcagg cggcggcttc 120

tcagggcgct gccgtctggc ttcctgccct acacggcctc gtcggttccc tgggccgcgc 180

gcaggctcaa ggtgctaacg gggctggcct aggccgcagc ctgggcggga gcgcaaggcc 240

gcgctgggag ccgctgtcgg cccctcagcc cggcccgggg cgttcacgcc actctccgcc 300

```
cggagcttgg ccgcgtcccc ctgtcgcgga ggccgcggtc cgatctgagg gcgtcgttaa      360
tgtgagagcc cggttgggag gctccaaacc gggcgccttc ggggaaggcc cgagcgccgg      420
agcccctgcc cggagcccgc gccgcggaac cgccccgccc tgggccgcag cgcgccgggc      480
ttgcagctcc gcttggcccg gccgcagctc gggagtctgg gaggttcggg ctaggaaaga      540
gagaaggagg ccaggcggcc aaagttaggt cagtttgttg gtgggtcgtt tggaattgcg      600
ctgggtccta gcaaactgtc gccttgtgac tgcagagtca cacagcgg                   648
```

## Description

Field of the Invention

The present invention relates to methods and kits for the diagnosis of celiac disease. Therefore, the present invention is comprised within the technical field of the medicine, specifically in the technical field of clinical diagnosis, and more specifically in the diagnosis of patients who suffer celiac disease.

Background of the Invention

Celiac disease is an autoimmune disease that appears in people who are genetically predisposed and is characterized by chronic inflammation of the proximal part of the small intestine, caused by exposure to gliadin. Gliadin is one of the main components of gluten; therefore the only treatment as of today for celiac disease is the strict absence of gluten in the diet.

Symptoms of celiac disease include chronic diarrhea, delay in childhood growth and/or development, fatigue, skin rashes, weight loss, changes in mood (irritability, apathy, introversion, sadness), vomiting, abdominal distension, loss of appetite, fatigue, etc.

The prevalence of celiac disease in Europeans and their descendants is 1%, being more common in women at a 2:1 ratio. Celiac disease usually manifests during the stage of childhood, although it has been demonstrated that it may also manifest throughout stage of adulthood.

Patients treated with gluten-free diets show lower serum antigen activities against gluten. However, that low activity against gluten, after introducing diets of this type, may be a consequence of the catabolism of the already formed antibodies, as well as of a decrease in their synthesis due to lower antigenic stimulation of gluten, and not necessarily due to an improvement in intestinal mucosa integrity.

Self-diagnosed gluten sensitivity and a self-imposed gluten-free diet are phenomena that are on the rise. Nevertheless, 10% of the general population and up to 12 or 13% in countries such as Italy or United Kingdom report undiagnosed gluten sensitivity, despite the fact that the prevalence of CD is in principle much lower. In this context, a less invasive and more efficient diagnostic tool than provocation with gluten and the subsequent biopsy would be an enormous clinical breakthrough.

The current strategy for the diagnosis of celiac disease is fundamentally based on demonstrating gluten-dependent enteropathy, for which invasive methods (biopsies) are still preferably used, although serum markers such as anti-gliadin antibodies are also analyzed. However, there are still no reliable analytical methods for following up on the effectiveness of the proposed treatment, since the histological analysis of duodenal biopsies is not feasible as a routine monitoring method for analyzing remission and treatment efficacy.

The determination of the levels of antibodies against other food antigens, as well as intestinal permeability measurements has been proposed as alternative methods of diagnosis of celiac disease. These methods would have as an advantage the absence of requiring biopsy samples.

Current analytical methods for celiac disease based on the histological analysis of duodenal biopsies do not allow adherence to a gluten-free diet by celiac patients to be evaluated either. It has been suggested that the analysis of the quantitative and qualitative changes in antibodies related to celiac disease may be useful in monitoring adherence to treatment with a gluten-free diet, and in fact, some of the antibodies analyzed to that end are the anti-transglutaminase (anti-tTG) and anti-deaminated gliadin (AGA) antibodies. However, these analyses do not necessarily reflect the actual degree of intestinal integrity, since a decrease in those antibodies may be a consequence of an increase in their catabolism or a decrease in their synthesis due to lower antigenic stimulation of gluten in gluten-free diets.

It has been described that the detection of tetramers HLA-DQ-gluten in blood could be used as a method of diagnosis of CD in the absence of gluten in the diet, with a 90% sensitivity and a 93% specificity (Sarna VK et al., Gastroenterology. 2018;154:886-96)

Therefore, in view of the state of the art, there is still a need to develop methods which allow both the diagnosis of celiac disease, the reliability of which is greater or at least provide more information than what the methods described to date do.

Summary of the Invention

In a first aspect, the invention relates to an *in vitro* method for the diagnosis of celiac disease in a subject suspected of suffering celiac disease which comprises quantifying in a sample from said subject the content of:

(i) the mRNA encoding isoform 2 of the UBE2L3 gene, and/or
(ii) the total mRNA of the UBE2L3 gene

wherein:

i) a low level of the mRNA encoding isoform 2 of the UBE2L3 gene with respect to a reference value,
ii) a high level of the total mRNA of the UBE2L3 gene with respect to a reference value, and/or
iii) a high relative content of the mRNA encoding isoform 2 of the UBE2L3 gene with respect to the total mRNA of the UBE2L3 gene with respect to a reference value is indicative that the subject has celiac disease.

In a second aspect, the invention relates to a kit for putting into practice the method of the first aspect of the invention comprising:

(i) A probe specifically hybridizing with exon 4 of the UBE2L3 gene,
(ii) A probe specifically hybridizing with exon 5 of the UBE2L3 gene, and optionally,
(iii) A probe specifically hybridizing with a constitutive expression gene

wherein components (i) and (ii) constitute at least 1% of the total of the probes present in the kit.

In a third aspect, the invention relates to a method for the diagnosis and treatment of a subject comprising:

(i) diagnosing in said subject the presence of celiac disease by means of the *in vitro* method of diagnosis of the invention;
(ii) administering to the subject diagnosed with celiac disease in step (i) a suitable treatment for said disease.

Brief Description of the Figures

Figure 1: Genomic location of the isoforms of the UBE2L3 gene in chromosome 22 (arrow indicating variant 2; numbers 4 and 5 indicating the fourth and fifth exons, respectively). The diagram shows the SNP prioritized by means of Mendelian randomization (MR) (rs5754217) and the Illumina expression microarray probes performed in patients with CD in a gluten-free diet (ILMN_1796830 and ILMN_1677877).
Figure 2: A. Expression in units of intensity reported by the two Illumina probes located in the UBE2L3 gene in controls (circles) and celiac patients with a gluten-free diet (squares). B: Relative expression between variant 2 and the remaining isoforms of the UBE2L3 gene calculated from the units of intensity reported by the Illumina probes independently.
Figure 3: ROC curve with the score of the relative expression of UBE2L3 as a binary classifier (CD in gluten-free diet/control) .

Detailed Description of the Invention

As shown in the examples of the application, the authors of the present invention have demonstrated that a decrease in the expression of isoform 2 of the UBE2L3 gene with respect to a control group is indicative of celiac disease, with a high specificity and sensitivity (AUC=0.997). Additionally, an increase in the expression of the total isoforms of the UBE2L3 gene with respect to a control group is indicative of celiac disease. Likewise, an increase in the relative expression of isoform 2 of the UBE2L3 gene in relation to the expression of all the isoforms of the gene, with respect to a control group, is also an indicator of celiac disease. In this case, the specificity of the method of diagnosis is 100% (AUC = 1). The extremely high predictive potential of the score of the relative expression of *UBE2L3* puts it in a privileged position for developing a diagnostic test in peripheral blood that could eliminate provocations with gluten from the diagnosis of CD in the absence of gluten in the diet.

***Methods of diagnosis***

Therefore, based on the findings that were made, in a first aspect the invention relates to an *in vitro* method for the diagnosis of celiac disease in a subject suspected of suffering celiac disease which comprises quantifying in a sample from said subject the content of:

(i) the mRNA encoding isoform 2 of the UBE2L3 gene, and/or
(ii) the total mRNA of the UBE2L3 gene

wherein:

- a low level of the mRNA encoding isoform 2 of the UBE2L3 gene with respect to a reference value,
- a high level of the total mRNA of the UBE2L3 gene with respect to a reference value, and/or
- a high relative content of the mRNA encoding isoform 2 of the UBE2L3 gene with respect to the total mRNA of the UBE2L3 gene with respect to a reference value is indicative that the subject has celiac disease.

As it is used herein, the term "diagnosis" refers both to the process of trying to determine and/or identify a possible disease in a subject, i.e., the method of diagnosis, and the opinion reached by this process, i.e., the diagnosis opinion. As such, the attempt to classify the condition of an individual in different separate categories which allow medical decisions to be made concerning the treatment and prognosis may also be considered.

As one skilled in the art will understand, such diagnosis may not be correct for 100% of the subjects to be diagnosed, although it is preferable that it is. However, the term requires a statistically significant part of the subjects to be identified as suffering a disease or being predisposed to same. One skilled in the art can determine if a result is statistically significant using different well-known statistical evaluation tools, for example, by means of the determination of confidence intervals, the determination of the p-value, the Student's t test, the Mann-Whitney test, etc. (see Dowdy and Wearden, 1983). The preferred confidence intervals are at least 50%, at least 60%, at least 70%, at least 80%, at least 90%, or at least 95%, or at least 99%. The p-values are preferably 0.05, 0.025, 0.001, or less.

The expression "method of diagnosis" to which reference is made according to the present invention means that the method may essentially consist of the aforementioned steps or may include additional steps.

As it is used herein, the expression "diagnosis *in vitro"* relates to a method of diagnosis that is not applied to a human or animal body.

The term "celiac disease" in the present invention refers to an autoimmune pathology characterized by chronic inflammation of the proximal part of the small intestine or jejunum, caused by exposure to gliadin, which is one of the components of gluten. Celiac disease is a permanent intolerance to gluten, in which an inflammatory immune reaction in the intestinal mucosa which hinders the absorption of nutrients is characteristic.

Gluten is a protein present in grains such as wheat *(Triticum spp),* barley *(Hordeum vulgare),* rye *(Secale cereale),* triticale (Triticosecale, grain coming from the cross between wheat and rye), kamut *(Triticum turgidum),* spelt *(Triticum spelta),* and possibly oat *(Avena* spp), but absent in rice *(Oryza sativa)* and corn *(Zea mays).* Gluten is made up of gliadin and glutenin, with gliadin being a prolamin-type glycoprotein. In the subject suffering celiac disease or a celiac patient, the intake of gliadin causes the tissue transglutaminase enzyme to modify said protein and the immune system brings about a cross-reaction against the small intestine, causing an inflammatory reaction which leads to atrophy of the villi covering the intestine and interference in the absorption of nutrients.

The development of celiac disease is determined both by environmental factors (food) and genetic factors. Therefore, celiac disease also implies a genetic predisposition since most celiac patients have the human leukocyte antigen (HLA), types DQ2 (HLA-DQ2) and DQ8 (HLA-DQ8) (May-Ling J. et al. 2010 Immunogenetics 62:641-651).

As it is used in the invention, the term "subject" refers to all animals classified as mammals and includes, but is not restricted to, domestic and farm animals, primates, and humans, for example, human beings, non-human primates, cows, horses, pigs, sheep, goats, dogs, cats, or rodents. Preferably, the subject is a male or female human being of any age or race.

The expression "comprising" intends to include, but without limitation, whatever follows the expression "comprising". Therefore, use of the expression "comprising" indicates that the mentioned elements are necessary or mandatory, but that other elements are optional and there is the possibility that they may or may not be present.

In relation to a nucleic acid sequence, "quantify" refers to the use of any method for studying the amount of a particular nucleic acid sequence, including, without limitation, methods for determining the number of copies of a nucleic acid sequence or for determining the change in the amount of copies of the nucleic acid sequence over time, or determining the relative concentration of a sequence when compared with another sequence.

Virtually any conventional method can be used in the framework of the invention for detecting and quantifying the levels of mRNA or of its corresponding cDNA. By way of nonlimiting illustration, the levels of mRNA encoded by said genes can be quantified by using conventional methods, for example, methods comprising amplification of the mRNA and quantification of the amplification product of said mRNA, such as electrophoresis and staining, or alternatively, by Northern blot and using suitable probes, using probes specific for the mRNA of the genes of interest or the corresponding cDNA, S1 nuclease mapping, RT-PCR, hybridization, microarrays, etc. Similarly, the levels of cDNA corresponding to said mRNA can also be quantified by using conventional techniques; in this case, the method of the invention includes a step of synthesizing the corresponding cDNA by means of reverse transcription (RT) of the corresponding mRNA followed by amplification and quantification of the amplification product of said cDNA. Conventional methods of quantifying expression levels can be found, for example, in Sambrook et al., 2001 "Molecular cloning: a Laboratory Manual", 3rd ed., Cold Spring Harbor Laboratory Press, N.Y., Vol.1-3.

In a preferred embodiment, the quantification of the product of the expression of the genes is performed by determining the level of mRNA derived from its transcription, where the analysis of the level of mRNA can be performed, by way of illustration and without limiting the scope of the invention, by means of polymerase chain reaction (PCR) amplification, combined reverse transcription-ligase chain reaction (RT-LCR), combined reverse transcription-polymerase chain reaction (RT-PCR), combined quantitative reverse transcription-polymerase chain reaction (qRT-PCR), or any other method of nucleic acid amplification; DNA microarrays produced with oligonucleotides deposited by any mechanism; DNA microarrays produced with oligonucleotides synthesized *in situ* by means of photolithography or by any other mechanism; hybridization *in situ* using specific probes labeled with any labeling method; by means of electrophoresis gels; by means of membrane transfer and hybridization with a specific probe; by means of NMR or any other imaging diagnostic technique using paramagnetic nanoparticles or any other type of detectable nanoparticles functionalized with antibodies or by any other means.

Additionally, the method of the invention may include performing an extraction step for the purpose of obtaining total RNA, which can be done by means of conventional techniques (Chomczynski et al., Anal. Biochem., 1987, 162:156; Chomczynski P., Biotechniques, 1993, 15:532).

As it is used in the present invention, the term "sample" refers to a sample, obtained from the subject under study (unless otherwise indicated), such as a blood or serum sample obtained from said subject. In a particular embodiment, said blood sample comprises peripheral blood. The term "peripheral blood" relates to the volume of circulating blood away from the heart, that is, the blood that circulates through the body of a subject. The blood sample can be obtained by conventional methods known to one skilled in the art. As it is used in the present invention, the term "serum" refers to the component of the blood resulting after clotting thereof and removal of the resulting clot. Methods of obtaining blood samples from a subject are widely described in the state of the art, as are methods of obtaining serum from blood samples.

The term "messenger RNA" or "mRNA" refers to RNA without introns and it may or may not be translated into a polypeptide.

The term "cDNA" refers to a nucleotide sequence complementary to an mRNA sequence.

The term "isoform" or "variant" refers to all the transcribed types of RNA of a given gene which, when collectively processed by means of alternative splicing or alternative processing, encode plural protein isoforms. The term "alternative splicing" or "alternative processing", refers to all the types of RNA processing which lead to the expression of plural protein isoforms of a single gene. Some genes, generally eukaryotic genes, are first transcribed as long mRNA precursors which are then shortened by means of a series of processing steps to produce the mature mRNA molecule. Alternative splicing of pre-mRNA is a post-transcriptional process that allows the production of different mRNAs from a single gene with the potential to expand protein structure and diversity. Alternative splicing may also introduce or eliminate regulating elements to affect mRNA translation, location, or stability. More than 70% of human genes may experience alternative splicing with many genes being capable of producing dozens and even hundreds of different isoforms. One of these steps is RNA splicing, in which the intron sequences are eliminated from the mRNA precursor. Once cell can bind the primary transcript in different ways, creating different "splicing variants" and, therefore, creating different polypeptide chains of the same gene or of the same mRNA molecule. Splicing variants may include, for example, exon insertions, exon extensions, exon truncations, exon deletions, alternatives in 5' untranslated region and alternatives in 3' untranslated region. Splicing variants are unpredictable, since the cell may bind the primary transcript in different ways, creating different polypeptide chains of the same gene or of the same mRNA molecule.

The term UBE2L3 refers to the UBE2L3 gene, which is found in chromosome 22 and encodes for the ubiquitin conjugating enzyme E2 L3. The UBE2L3 is also known as UBCH7, L-UBC, UbcM4, E2-F1, or UBCE7. The alternative processing of the UBE2L3 gene gives rise to 5 mRNA isoforms or variants. Said isoforms can be defined by their corresponding NCBI accession number and by the number and specific sequences of exons, as indicated in the following table:

Table 1. Isoforms of the UBE2L3 gene, corresponding sequences, accession number, and exons.

| ISOFORM | POSITION IN THE SEQUENCE | | | | |
|---|---|---|---|---|---|
| | Exon 1 | Exon 2 | Exon 3 | Exon 4 | Exon 5 |
| 1 NM_003347.3 04/12/2019 | 1-225 SEQ ID NO: 1 | 226-321 SEQ ID NO: 2 | 322-508 SEQ ID NO: 3 | ---- | 509-3028 SEQ ID NO: 4 |
| 2 NR_028436 24/02/2019 | 1-225 SEQ ID NO: 1 | 226-321 SEQ ID NO: 2 | 322-508 SEQ ID NO: 3 | 509-617 SEQ ID NO: 5 | 618-3137 SEQ ID NO: 4 |

(continued)

| ISOFORM | POSITION IN THE SEQUENCE | | | | |
|---|---|---|---|---|---|
| | Exon 1 | Exon 2 | Exon 3 | Exon 4 | Exon 5 |
| 3 NM_001256356. 1 24/02/2019 | 1-225 SEQ ID NO: 1 | ---- | 226-412 SEQ ID NO: 3 | ---- | 413-2932 SEQ ID NO: 4 |
| 4 NM_001256355. 24/02/2019 | 1-204 SEQ ID NO: 6 | 205-300 SEQ ID NO: 2 | 301-487 SEQ ID NO: 3 | ---- | 488-3007 SEQ ID NO: 4 |
| 5 NR_046082.1 24/02/2019 | 1-648 SEQ ID NO: 7 | ---- | 745-931 SEQ ID NO: 3 | ---- | 932-3451 SEQ ID NO: 4 |

Therefore, as it is used in the context of the present invention, the term isoform 2 of the UBE2L3 gene refers to the sequence identified with NCBI accession number NR_028436 of 24 February 2019. Said sequence corresponds to a non-encoding isoform of the gene and comprises exons 1, 2, 3, 5, and 4 defined respectively by sequences SEQ ID NO: 1, SEQ ID NO: 2, SEQ ID NO: 3, SEQ ID NO: 4, and SEQ ID NO: 5, as specified in Table 1.

Total mRNA refers to the sum of all the isoforms of the UBE2L3 gene, i.e., the isoforms defined by NCBI accession numbers NM_003347.3, NR_028436, NM_001256356.1, NM_001256355.1, and NR_046082.1 of 24 February 2019.

As previously indicated, levels of mRNA may be measured and quantified by various methods well known to those skilled in the art, including the use of commercially available equipment and reagents. Quantification of the expression levels of the UBE2L3 gene can be performed from the RNA resulting from the transcription of said genes (mRNA), or alternatively from the complementary DNA (cDNA) of said genes. Therefore, in a particular embodiment, the quantification of the expression levels of the UBE2L3 gene comprises the quantification of the messenger RNAs (mRNAs) of said genes, or a fragment of said mRNAs, the complementary DNA (cDNA) of said genes, or a fragment of said cDNA, or the mixtures thereof.

The determination of the expression levels of the UBE2L3 gene, whether by means of quantifying mRNA or of the cDNA of isoform 2, or by means of quantifying mRNA or total cDNA, needs to be compared with the reference values.

As it used in the present description, the term "reference value", "cut-off", or "reference amount" refers to any value or range of values derived from the quantification of the product of the expression of the UBE2L gene in a biological sample, or in a collection of biological samples, from individuals who do not suffer celiac disease. The reference amount must be measured in the same way and be obtained in the same type of isolated biological sample as the amount measured in the analyzed subject.

As is used in the description, the term "comparison" refers but is not limited to the comparison of the amount of the product of the expression of the UBE2L3 gene or of any of the isoforms thereof with a reference value. The comparison described in the method of the present invention can be performed manually or be computer-assisted.

In the present invention, the reference value corresponds to the mean value of the expression levels of the UBE2L3 gene or of any of the isoforms thereof, measured in a sample from non-celiac subjects. Once this mean value has been established, the level of this marker expressed in samples from celiac subjects can be compared with this mean value and thereby be assigned to the "low" or "high" expression level.

Due to the variability between subjects (for example, aspects relating to age, race, etc.) it is very difficult (if not virtually impossible) to establish absolute reference expression values of the UBE2L3 gene or of any of the isoforms thereof. Therefore, in a particular embodiment, the "high" or "low" reference expression values of the UBE2L3 gene or of any of the isoforms thereof are determined by calculating the percentiles by conventional means which implies testing a group of isolated samples from normal subjects (i.e., people without celiac disease) for the expression levels of UBE2L3 or of any of the isoforms thereof. The "low" levels of UBE2L3 or of any of the isoforms thereof can then preferably be assigned to samples in which the expression levels of UBE2L3 or of any of the isoforms thereof are equal to or less than $50^{th}$ percentile in the normal population, including, for example, expression levels equal to or less than $60^{th}$ percentile in the normal population, equal to or less than $70^{th}$ percentile in the normal population, equal to or less than $80^{th}$ percentile in the normal population, equal to or less than $90^{th}$ percentile in the normal population, and equal to or less than $95^{th}$ percentile in the normal population. The "high" levels of UBE2L3 or of any of the isoforms thereof can then preferably be assigned to samples in which the expression levels of UBE2L3 or of any of the isoforms thereof are equal to or exceed $50^{th}$ percentile in the normal population, including, for example, expression levels equal to or above $60^{th}$ percentile in the normal population, equal to or above $70^{th}$ percentile in the normal population, equal to or above $80^{th}$ percentile in the normal population, equal to or above 90th percentile in the normal population, and equal to or above 95th percentile in the normal population.

Therefore, according to the method of the invention, celiac disease can be diagnosed based on the following indicators:

- a low level of the mRNA of isoform 2 of the UBE2L3 gene with respect to a reference value;
- a high level of the total mRNA of the UBE2L3 gene with respect to a reference value; and
- a high level of the relative content of mRNA of isoform 2 of the UBE2L3 gene with respect to the total of the mRNA of the UBE2L3 gene with respect to a reference value.

As it is used in the context of the method of the invention, "relative expression" or "relative content" is the amount of expressed mRNA of isoform 2 of the UBE2L3 gene with respect to the amount of total mRNA of the UBE2L3 gene, both amounts expressed in the same units depending on the method used for the quantification of said mRNA according to the techniques known in the state of the art, or as indicated above, of the corresponding cDNA. The reference value in this case is established by means of calculating the relative expression of isoform 2 of the UBE2L3 gene with respect to the total mRNA of a control group who do not suffer celiac disease.

In a particular embodiment, the reference value is selected from:

(i) the content of mRNA encoding isoform 2 of the UBE2L3 gene in a subject who does not have celiac disease or the mean value of the content of mRNA encoding isoform 2 of the UBE2L3 gene in a population of subjects who do not have celiac disease,
(ii) the content of total mRNA of the UBE2L3 gene in a subject who does not have celiac disease or the mean value of the content of total mRNA of the UBE2L3 gene in a population of subjects who do not have celiac disease, and/or
(iii) the relative content of the mRNA encoding isoform 2 of the UBE2L3 gene with respect to the total mRNA of the UBE2L3 gene in a subject who does not have celiac disease or the mean value of the relative content of the mRNA encoding isoform 2 of the UBE2L3 gene with respect to the total mRNA of the UBE2L3 gene in a population of subjects who do not have celiac disease.

In another particular embodiment, the relative content of the mRNA encoding isoform 2 of the UBE2L3 gene with respect to the total mRNA of the UBE2L3 gene is determined by means of formula (I):

$$\mathrm{SCORE} = 2^{(\text{amount of mRNA of isoform 2 of UBE2L3} - \text{ amount of total mRNA of UBE2L3})}$$

In a preferred embodiment, the levels of mRNA of isoform 2 of the UBE2L3 gene or of the total mRNA of the UBE2L3 gene are normalized with respect to the level of a constitutive expression gene.

To normalize the expression values of the mRNA between different samples, it is possible to compare the expression levels of the mRNA of interest in the samples to be tested with the expression of a control RNA corresponding to a constitutive expression gene. As it is used herein, a "control RNA" refers to an RNA the expression levels of which do not change or only change by limited amounts in cells of celiac subjects compared with non-celiac subjects. Preferably, the control RNA is mRNA derived from maintenance genes and encoding proteins that are constitutively expressed and carry out essential cell functions. Examples of maintenance genes for use in the present invention include β-2-microglobulin, ubiquitin, 18-S ribosomal protein, cyclophilin, GAPDH, and actin. In a preferred embodiment, the control RNA is GAPDH mRNA. In one embodiment, quantification of relative gene expression is calculated according to the comparative Ct method using GAPDH as endogenous control and commercial RNA controls as calibrators. The final results are determined according to formula 2-(ΔCt of the sample-ΔCt of the calibrator), where the ΔCT values of the calibrator and sample are determined by subtracting the CT value of the target gene from the value of the GAPDH gene.

In a particular embodiment, the sample comprises peripheral blood mononuclear cells (PBMCs).

Peripheral blood mononuclear cells are characterized by having a single round nucleus. These cells consist of lymphocytes (cells T, cells B, cells NK) and monocytes. In human beings, lymphocytes make up most of the PBMC population, followed by monocytes, and only a small percentage of dendritic cells.

PBMC extraction methods are well known in the state of the art and are part of common research and clinical laboratory techniques. For example, these cells can be extracted by means of density-gradient centrifugation (J. W. Mannhalter et al., Clin. Immunol. Immunopathol. 1986 38, 390 - 397).

In a particular embodiment, the mRNA encoding isoform 2 of the UBE2L3 gene is determined by using a probe specifically hybridizing with an exon 4 region of the UBE2L3 gene and/or the total mRNA of the UBE2L3 gene is determined by using a probe specifically hybridizing with an exon 5 region of the UBE2L3 gene.

"Probe" is defined as a sequence of nitrogenous bases of adenine (A), cytosine (C), guanine (G), thymine (T), or uracil (U), arranged in a linear polymer that is completely or partially complementary (Watson and Crick type pairings: A-T/U or G-C) to another one present in the sample to be analyzed. Such probes can be nucleic acid (DNA, RNA) probes, peptide nucleic acid (PNA) probes, or other synthetic nucleic acid probes capable of hybridizing with other nucleic acids by means of Watson and Crick type pairings.

Said probes can be or a DNA fragment amplified by PCR, or an oligomer with nitrogenous bases (which is synthetic or is obtained by enzymatic digestion or other chemical or physical means of an existing polymer).

In a particular embodiment of the invention, the probes are synthetic oligonucleotides with modifications at one or at both ends to make immobilization on solid supports easier.

The probes are designed and constructed such that their base sequence is unique for the variety it represents, and such that discrepancies (non-complementary nucleotides) between two or more variants are usually in the central areas of the oligonucleotides.

The length of the oligonucleotides may vary between 5 or more bases, the length most suitable for discriminating between two very similar but not identical nucleic acid sequences preferably being between 11 and 30 nucleotides.

The term "specific hybridization" refers to the formation of hybrids between a polynucleotide probe and a specific target polynucleotide (for example, the mRNA of the UBE2L3 gene or any of the isoforms or exons thereof) in which the probe preferably hybridizes with the specific target polynucleotide and does not substantially hybridize with polynucleotides consisting of sequences which are not substantially identical to the target polynucleotide. However, those skilled in the art will recognize that the minimum length of a polynucleotide required for a specific hybridization with a target polynucleotide will depend on several factors, for example: the G/C content, the positioning of non-matching bases (if there are any), the degree of singularity of the sequence compared with the population of target polynucleotides, and the chemical nature of the polynucleotide (for example, the main methylphosphonate or phosphorothiolate chain), among others.

In a particular embodiment the probes have an identity of at least 80%, more preferably at least 90%, even more preferably at least 95%, even much more preferably at least 98%, and particularly 100%, with a fragment of the sequences complementary to the sequences defined by NCBI accession numbers described in Table 1, as well as of exons defined by sequences SEQ ID NO: 1-7.

The terms "complementary" and "substantially complementary" refer to the base pairing or hybridization between two nucleotides or nucleic acid molecules, such as for example, between the two strands of a double-stranded DNA molecule or between an oligonucleotide primer and a primer binding site in a single-stranded nucleic acid to be sequenced or amplified. Complementary nucleotides are, generally, A and T (or A and U), and C and G. Two single-stranded RNA or DNA molecules are said to be substantially complementary when the nucleotides of one strand, optimally aligned and compared with suitable nucleotide insertions or deletions, are paired with at least about 80% of the nucleotides of the other strand, normally at least about from 90% to 95%, and more preferably from 98 to 100%. Substantial complementarity exists where one strand of RNA or DNA hybridizes under selective hybridization conditions with its complementary RNA or DNA. Selective hybridization will normally occur when there is at least 65% complementarity across a section of at least 14 to 25 nucleotides, preferably at least about 75%, and more preferably at least 90% complementarity. See, for example, M. Kanehisa (1984) Nucleic Acids Res.12:203.

As shown in Table 1 mentioned above, isoform 2 of the UBE2L3 gene is the only one of the isoforms of the gene containing exon 4. As it is used in the present invention, exon 4 refers to the sequence comprised between positions 509 and 617 of the nucleotide sequence defined by NCBI accession number NR _028436 of 24 February 2019 and referred to in the present invention as SEQ ID NO: 4.

```
AA ACTTCAGCGT TCCCAATTAT GGCTTCTCTC AGATCCAGCC TTGAAGTTCT
TTGACCTCCT CAATTCACAA CCTGTAGCTG ACTTTAGCCA CCCACAAGTA CAGAAAA
(SEQ ID NO: 4)
```

Said exon 4 is only present in isoform 2 of the UBE2L3 gene of the sequence defined by NCBI accession number NR_028436 of 24 February 2019.

As it is used in the present invention, exon 5 is defined by sequence SEQ ID NO: 5 and corresponds to the last exon of all the isoforms of the UBE2L3 gene, as shown in Table 1 of the present description.

In another particular embodiment, the total mRNA of the UBE2L3 gene is determined by using a probe specifically hybridizing with a region of exon 3 of the UBE2L3 gene. In this case, exon 3 is defined by sequence SEQ ID NO: 3 as specified in Table 1. In another embodiment, the total mRNA of the UBE2L3 gene is determined by means of any probe binding specifically to a region common to all the isoforms of the UBE2L3 gene.

In a preferred embodiment, the quantification of mRNA encoding isoform 2 of the UBE2L3 gene is carried out by means of quantifying cDNA the sequence of which is defined by NCBI accession number NR_028436.2 of the version from 4 December 2018.

In another particular embodiment, the quantification of total mRNA of the UBE2L3 gene is carried out by means of quantifying the sum of the levels of mRNA of isoforms 1 to 5 of the UBE2L3 gene with NCBI accession numbers NM_003347.3, NR_028436, NM_001256356.1, NM_001256355.1, and NR_046082.1 of 24 February 2019.

In another preferred embodiment, the quantification of mRNA is performed by means of an expression microarray.

A "microarray" is a multiplex technology typically using an ordered series of thousands of nucleic acid probes for hybridizing, for example, with a sample of cDNA or cRNA under astringent hybridization conditions. Hybridization of the probe with the nucleic acid to be identified generates a signal that can be detected and/or quantified. By way of example, the probes can be labeled with a fluorophore, silver, or chemiluminescence. The signal emitted upon hybridizing therefore allows determining the relative abundance of sequences of the nucleic acid to be detected. The probes are connected to a solid support by a covalent bond with a chemical matrix (by means of epoxy silane, amino silane, lysine, polyacrylamide, or others). As it is used in the present invention, the term "solid support" relates to a wide range of materials, for example, but without limitation, ion exchange or adsorption resin, glass, plastic, latex, nylon, gel, cellulose esters, paramagnetic spheres, silicon, microscopic beads, or the combination of some of them. Various microarrays are commercially available including those manufactured, for example, by Affymetrix, Inc. and Illumina, Inc.

The "astringent hybridization conditions" normally include concentrations of salt of at least 1 M, more generally less than around 500 mM, and preferably less than about 200 mM. Hybridization temperatures may be as low as 5°C, but are normally greater than 22°C, more normally greater than about 30°C, and preferably above about 37°C. The longest fragments may require higher hybridization temperatures for specific hybridization. Since other factors may affect the hybridization astringency, even the composition of the bases and length of the complementary strands, the presence of organic solvents and the degree of base unpairing, the combination of parameters is more important than the absolute measurement of any single parameter.

In the context of the method of the present invention, the detection of the content of isoform 2 of the UBE2L3 gene can be carried out using any probe capable of hybridizing with a region of the mRNA or cDNA of exon 4 (SEQ ID NO: 4). In another preferred embodiment, the detection of the total mRNA content of the UBE2L3 gene is carried out using a probe capable of hybridizing with a region of the mRNA or cDNA of exon 5 (SEQ ID NO: 5), of exon 3 (SEQ ID NO: 3), or of any region common to 5 isoforms of the UBE2L3 gene. In a specific embodiment, the probe used for the recognition and quantification of exon 4 is ILMN_1796830 by *Illumina.* In another particular embodiment, probe ILMN_1677877 by *Illumina* is used for the recognition and quantification of exon 5 of the UBE2L3 gene.

In another preferred embodiment, the method of diagnosis is applied to a subject suspected of suffering celiac disease who follows a substantially gluten-free diet.

A "gluten-free diet (GFD)" refers to the strict removal from food of all products containing or made with wheat, rye, barley, and oat, or any of their varieties and hybrids (spelt, dinkel wheat, kamut, triticale, etc.), and derivative products, avoiding inadvertent contaminations and any type of dietary transgressions.

"Substantially gluten-free" generally refers to foodstuffs and/or any component thereof which do not contain gluten and/or contain an acceptable amount of gluten for an applicable government distribution, a food regulating entity, a group of industries or the like to label them as "gluten-free". The US Food and Drug Administration (FDA) recognizes as "gluten-free" foodstuffs those which do not have: (1) an ingredient that is any type of wheat, rye, barley, or hybrids of these grains; (2) an ingredient derived from these grains and not processed for removing gluten; and (3) an ingredient derived from these grains that have been processed for removing gluten, if it results in the foodstuff containing 20 ppm or more of gluten. Other countries such as, for example, New Zealand and Australia, allow labeling a food "gluten-free" in foodstuffs having less than 3 ppm of gluten. A foodstuff which is substantially gluten-free may have a gluten content less than or equal to 20 parts per million (ppm), including 15 ppm, 10 ppm, 5 ppm, 3 ppm, 1 ppm, 0.5 ppm, 0.1 ppm, 0.05 ppm, 0 ppm, or any value or range between any two of these values (including the endpoints). Any of the foodstuffs, solid compositions, particulate compositions, dry compositions, or the like indicated as being gluten-free are recognized by those skilled in the art as optionally being substantially gluten-free.

### *Kit of the **invention***

In an additional aspect, the invention relates to a kit for putting into practice a method as defined in claims 1 to 10 comprising:

(i) A probe specifically hybridizing with exon 4 of the UBE2L3 gene,
(ii) A probe specifically hybridizing with exon 5 of the UBE2L3 gene, and optionally,
(iii) A probe specifically hybridizing with a constitutive expression gene

wherein components (i) and (ii) constitute at least 1% of the total of the probes present in the kit.

The kit is based on the predictive power of the method of diagnosis of the present invention. The reference value of the expression levels of the UBE2L3 gene or of any of the isoforms thereof can be determined before carrying out the method of the present invention. With the help of the kit, the expression of the UBE2L3 gene or of any of the isoforms thereof with respect to the control samples can be calculated. The control can thereby also be comprised in the kit.

The kit of the invention more preferably comprises the means necessary for quantifying the expression of the UBE2L3 gene or any of the isoforms thereof, and optionally for comparing the detected amount with a reference amount. Said kit may contain all those reagents necessary for analyzing the amount of the product of expression of the UBE2L3 gene as well as any of the isoforms thereof and any of the exons (defined by sequences SEQ ID NO: 1 to 7) by means of any of the methods known in the state of the art mentioned hereinabove. The kit may furthermore include, without any type of limitation, buffers, agents for preventing contamination, RNA degradation inhibitors, etc. Moreover, the kit may include all the supports and vessels necessary for implementing and optimizing it. Preferably, the kit further comprises instructions for carrying out the method of the invention.

More preferably, the kit or device of the invention comprises the primers and probes obtained from the sequences of the invention (Table 1). The kit may contain the probe/probes and primers useful for quantifying the expression of said gene, or any of the exons described in Table 1, as well as the combinations thereof.

In particular embodiments, the kit is selected from (a) a kit suitable for PCR, (b) a kit suitable for Northern blot, and (c) a kit suitable for microarray analysis. Any two or more of these embodiments may be combined, such that the kit may comprise, for example, both (a) and (c).

In the case of (a) a kit suitable for PCR, this PCR is normally the real-time quantitative PCR (RQ-PCR), a sensitive and reproducible technique for quantifying gene expression. In this case, it is desirable for the kit to additionally comprise primers and probes and oligonucleotide (s) of the kit. These reagents may optionally be comprised in the kit.

Northern blot implies the use of electrophoresis for separating the RNA samples by size and the subsequent detection with the oligonucleotide(s) (hybridization probe) complementary with (part of) the target sequence of the RNA of interest.

It is also possible for the oligonucleotide(s) to be immobilized on a (preferably solid) surface. In one of its embodiments, the kit comprises a microarray. An RNA or DNA microarray is an array on a solid substrate (normally a glass slide or a thin-film silicon cell) which evaluates large amounts of different RNA or DNA which are detectable by means of specific probes immobilized on a solid substrate. Each solid support contains a specific nucleic acid sequence, normally a DNA sequence, as probes (or indicators). Although the number of probes is not limited in any way, there is a preferred embodiment in which the microarray is customized for the methods of the invention. In one embodiment, said customized microarray comprises fifty probes or fewer, such as thirty probes or fewer, including twenty probes or fewer. The kit may contain hybridization, enhancing, blocking, washing buffers and solutions, and any component necessary for carrying out the method of diagnosis according to common practice.

In a particular embodiment, the kit of the invention additionally comprises one or more components selected from the group consisting of:

(i) Means for purifying RNA from a cell sample,
(ii) Means for reverse transcription of an mRNA preparation, and
(iii) Means for amplification of exons 4 and 5 of the UBE2L3 gene.

The kit of the invention could therefore include any means known in the state of the art for purifying RNA, such as RNA isolation based on extraction with phenol, precipitation by using chaotropic salt solutions, or adsorption in silica, among others, for converting mRNA to cDNA or for amplification of exons 4 and 5 of the UBE2L3 gene.

*Method of diagnosis and* treatment

In another aspect, the invention relates to a method for the diagnosis and treatment of a subject comprising:

(i) diagnosing in said subject the presence of celiac disease by means of the method according to claims 1 to 10, and
(ii) administering to the subject diagnosed with celiac disease in step (i) a suitable treatment for said disease.

Preferred embodiments of step (i) of diagnosing of the present aspect have been described in detail in the context of the method of diagnosis of the invention and are likewise applicable to the method of diagnosis and treatment.

In the context of the present invention, the expression "suitable treatment" primarily refers to following a gluten-free diet, as described above. Specific vaccines or enzyme treatments for the degradation and neutralization of gluten could further be used. Likewise, the use of medicinal products which help to maintain the intestinal barrier may be assessed. In the case of refractory celiac disease, treatment may imply the use of steroids.

In a particular embodiment, the treatment is a gluten-free diet.

The invention will be described below by means of the following examples which are merely illustrative and do not limit the scope of the invention.

## EXAMPLES

Methods

Generally, the clinical usefulness of genome-wide association studies (or GWAS) has been very limited, so it is necessary to develop strategies that allow identifying the truly important variation and translating those findings into applications. In turn, Mendelian randomization (MR) is a statistical method whereby genes can be prioritized using information about how they are related to genetic polymorphisms or adjacent SNPs and about how the SNPs are in turn associated with different phenotypes (Zhu Z et al., Nat Genet. 2016;48:481-7). MR therefore allows identifying genes mediating the SNP-disease association, imparting to it certain causality and a plausible mechanism.

For the purpose of prioritizing the causal genes involved in the associations identified in different GWAS studies performed in celiac disease (CD), SMR software was used to apply MR (Zhu Z et al., Nat Genet. 2016;48:481-7). More specifically, the results of the largest GWAS carried out in CD (9451 cases vs. 16434 controls) were analyzed (Dubois PC et al., Nat Genet. 2010;42:295-302) and crossed with whole-genome expression data in relation to genotype (expression quantitative trait loci or eQTLs) of the GTEx database (GTEx Consortium. Genetic effects on gene expression across human tissues. Nature. 2017;550:204-13) (p<1e-5), from blood (n=122) and small intestine (n=369). SMR software was also applied to the same GWAS data with DNA methylation as a possible mediator in the associations, using to that end the Lite version of the methylation quantitative trait loci (mQTL) database of McRae et *al.* (McRae A et *al.,* Identification of 55,000 Replicated DNA Methylation QTL. bioRxiv 166710) (p<1e-5; n=1,366), available on the SMR web page (https://cnsgenomics.com/software/smr/#DataResource). Moreover, the results obtained from the two independent experiments (SMR with eQTLs and mQTLs) were crossed for the purpose of finding SNPs associated with celiac disease by means of the expression of genes with changes in their methylation levels. Then the results obtained with blood expression data were replicated by means of the CAGE database eQTLs (Lloyd-Jones LR et al., Am J Hum Genet. 2017; 100:228-237) (p<1e-5; n= 2,765), also available on the web site mentioned above.

All the analyses were carried out with the SMR software default parameters, including the discarding of association signals derived from the linkage disequilibrium by means of the Heidi algorithm (p<0.01) and an empirical threshold value of p less than 1e-4 for the SMR test, due to the limited power of the study particularly derived from the sample size of the eQTL/mQTL studies used.

Finally, selected candidates were polled by means of prioritizing genes via MR into two completely independent databases of the whole-genome expression in peripheral blood mononuclear cells available at GEO (https://www.ncbi.nlm.nih.gov/geo/): an analysis in the Illumina HumanHT-12 V4.0 expression beadchip platform in 17 patients with CD on a gluten-free diet and 20 controls without intestinal inflammation (GSE113469) (Sangineto M et al., PLoS One. 2018;13:e0197915), and a study in another type of expression microarray-Affymetrix Human Genome U133A Array of 42 controls, 59 patients with Crohn's disease and 26 with ulcerative colitis (GSE3365) (Burczynski ME et al., J Mol Diagn. 2006;8:51-61). Comparisons were carried out by means of applying Mann-Whitney U tests, and Receiver Operating Characteristic (ROC) curves were constructed, using the expression levels of candidate genes and their combinations as binary classifiers. Namely, the relative expression of *UBE2L3* in CD was calculated from the intensity units of housekeeping gene GAPDH and from the probes available in the aforementioned Illumina array for UBE2L3 as indicated below:

$$2^{((UBE2L3.1 - UBE2L3.2)/GAPDH)}$$

where UBE2L3.1=ILMN_1677877 (exon 5 common to all the gene variants) and UBE2L3.2=ILMN_1796830 (exon 4 exclusive of non-encoding variant number 2).

## Results and discussion

MR analysis with GTEx small intestine eQTL data did not provide any significant result, whereas the same analysis with the data about blood from the same database resulted in the prioritization of three SNPs associated with CD by means of the expression of their adjacent genes *AHSA2, AC007278.2,* and *UBE2L3* (Table 2).

Table 2: Summary of results obtained from MR analysis in the celiac GWAS with GTEx blood eQTL data. In addition to the chromosomal location of each SNP and each gene, the size of the effect of each study used (b), as well as the p-value (p) thereof is shown.

| Gene | topSNP | topSNP_chr | topSNP_bp | b_GWAS | p_GWAS | b_eQTL | p_eQTL | b_SMR | p_SMR |
|---|---|---|---|---|---|---|---|---|---|
| *AHSA2* | rs4672441 | 2 | 61638952 | -0.126633 | 0.00000167 | 0.438683 | 9.51867E-38 | -0.288666 | 7.63033E-06 |
| *AC007278.2* | rs917997 | 2 | 103070568 | -0231905 | 597E-15 | 0.231665 | 1.48261E-15 | -1.00104 | 2,4163E-08 |
| *UBE2L3* | rs5754217 | 22 | 21939675 | 0.151862 | 0.000000692 | 0.162817 | 2.41949E-13 | 0.932718 | 4.09239E-05 |

However, when these results were crossed with those obtained from the MR of celiac GWAS with mQTLs, only SNP rs5754217 remained located in the first intron of *UBE2L3* in chromosome 22 (Figure 1). Furthermore, this SNP-gene binomial passed the SMR test again when eQTLs from CAGE blood instead of GTEx were used, such that the results could be replicated in a larger dataset (b=0.165547, standard error=0.0340045, p=1.13E-06) .

Moreover, when expression in CD patients with a gluten-free diet (n=17) was compared with the controls (n=20) of the expression dataset of Illumina in peripheral blood mononuclear cells, the two probes showed opposite but very significant behaviors (p<0.0001) (Figure 2A). Furthermore, the construction of a relative expression score (as determined in the Methods) gave rise to an even greater difference between CD and controls (Figure 2B).

Finally, ROC curves were constructed to assess the potential diagnosis of the expression of the different exons of UBE2L3 separately and of the relative expression. It was observed that although the independent expression of exons is an efficient classifier (area under the curve - AUC=0.997 per probe), the relative expression of *UBE2L3* has a surprising specificity and sensitivity of 100%, and therefore an AUC value=1 (Figure 3). Furthermore, this discrimination capacity seems to be CD-specific since it was not observed in a public and independent dataset of expression in peripheral blood mononuclear cells of Crohn's disease and ulcerative colitis for any of the probes found in candidate UBE2L3. Nevertheless, the resulting AUC values were 0.528-0.699 and 0.452-0.752 for Crohn's and ulcerative colitis, respectively.

The extremely high predictive potential of the relative expression score of UBE2L3 puts it in a place of privilege for the development of a diagnostic test in peripheral blood which could eliminate provocations with gluten in the diagnosis of CD in the absence of gluten in the diet.

## Claims

1. An *in vitro* method for the diagnosis of celiac disease in a subject suspected of suffering celiac disease which comprises quantifying in a sample from said subject the content of:

   (i) the mRNA encoding isoform 2 of the UBE2L3 gene, and/or
   (ii) the total mRNA of the UBE2L3 gene,

   wherein:

   - a low level of the mRNA encoding isoform 2 of the UBE2L3 gene with respect to a reference value,
   - a high level of the total mRNA of the UBE2L3 gene with respect to a reference value, and/or
   - a high relative content of the mRNA encoding isoform 2 of the UBE2L3 gene with respect to the total mRNA of the UBE2L3 gene with respect to a reference value is indicative that the subject has celiac disease.

2. The method according to claim 1, wherein the reference value is selected from:

   (i) the content of mRNA encoding isoform 2 of the UBE2L3 gene in a subject who does not have celiac disease or the mean value of the content of mRNA encoding isoform 2 of the UBE2L3 gene in a population of subjects who do not have celiac disease,
   (ii) the content of total mRNA of the UBE2L3 gene in a subject who does not have celiac disease or the mean value of the content of total mRNA of the UBE2L3 gene in a population of subjects who do not have celiac disease, and/or
   (iii)the relative content of the mRNA encoding isoform 2 of the UBE2L3 gene with respect to the total mRNA of the UBE2L3 gene in a subject who does not have celiac disease or the mean value of the relative content of the mRNA encoding isoform 2 of the UBE2L3 gene with respect to the total mRNA of the UBE2L3 gene in a population of subjects who do not have celiac disease.

3. The method according to claim 1, wherein the relative content of the mRNA encoding isoform 2 of the UBE2L3 gene with respect to the total mRNA of the UBE2L3 gene is determined by means of formula (I):

$$\text{SCORE} = 2^{(\text{amount of mRNA of isoform 2 of UBE2L3} - \text{ amount of total mRNA of UBE2L3})}$$

4. The method according to any of claims 1 to 3, wherein the levels of mRNA of isoform 2 of the UBE2L3 gene or of the total mRNA of the UBE2L3 gene are normalized against the level of a constitutive expression gene.

5. The method according to any of claims 1 to 4, wherein the sample in which the mRNA is quantified comprises peripheral blood mononuclear cells.

6. The method according to any of claims 1 to 5, wherein the mRNA encoding isoform 2 of the UBE2L3 gene is determined by using a probe specifically hybridizing with an exon 4 region of the UBE2L3 gene and/or wherein the total mRNA of the UBE2L3 gene is determined by using a probe specifically hybridizing with an exon 5 region of the UBE2L3 gene.

**7.** The method according to any of claims 1 to 5, wherein the quantification of mRNA encoding isoform 2 of the UBE2L3 gene is carried out by means of quantification of cDNA the sequence of which is defined by NCBI accession number NR_028436.2 of the version from 4 December 2018.

**8.** The method according to any of claims 1 to 7, wherein the quantification of total mRNA of the UBE2L3 gene is carried out by means of quantifying the sum of the levels of mRNA of isoforms 1 to 5 of the UBE2L3 gene with NCBI accession numbers NM_003347.3 of 11 November 2018, NM_001256, NR_028436 of 4 December 2018, NM_001256356.1 of 11 November 2018, NM_001253655.4 of 11 November 2018, and NR_046082.1 of 4 December 2018 respectively.

**9.** The method according to any of claims 1 to 4, wherein the quantification of mRNA is performed by means of an expression microarray.

**10.** The method according to any of claims 1 to 9, wherein the subject suspected of suffering celiac disease follows a substantially gluten-free diet.

**11.** A kit for putting into practice a method as defined in claims 1 to 10 comprising:

(i) A probe specifically hybridizing with exon 4 of the UBE2L3 gene,
(ii) A probe specifically hybridizing with exon 5 of the UBE2L3 gene, and optionally,
(iii)A probe specifically hybridizing with a constitutive expression gene

wherein components (i) and (ii) constitute at least 1% of the total of the probes present in the kit.

**12.** The kit according to claim 11, further comprising one or more components selected from the group consisting of:

(i) Means for purifying RNA from a cell sample,
(ii) Means for reverse transcription of an mRNA preparation, and
(iii)Means for amplification of exons 4 and 5 of the UBE2L3 gene.

**13.** A method for the diagnosis and treatment of a subject comprising:

(i) diagnosing in said subject the presence of celiac disease by means of the method according to claims 1 to 10, and
(ii) administering to the subject diagnosed with celiac disease in step (i) a suitable treatment for said disease.

**14.** The method according to claim 13, wherein the treatment is a gluten-free diet.

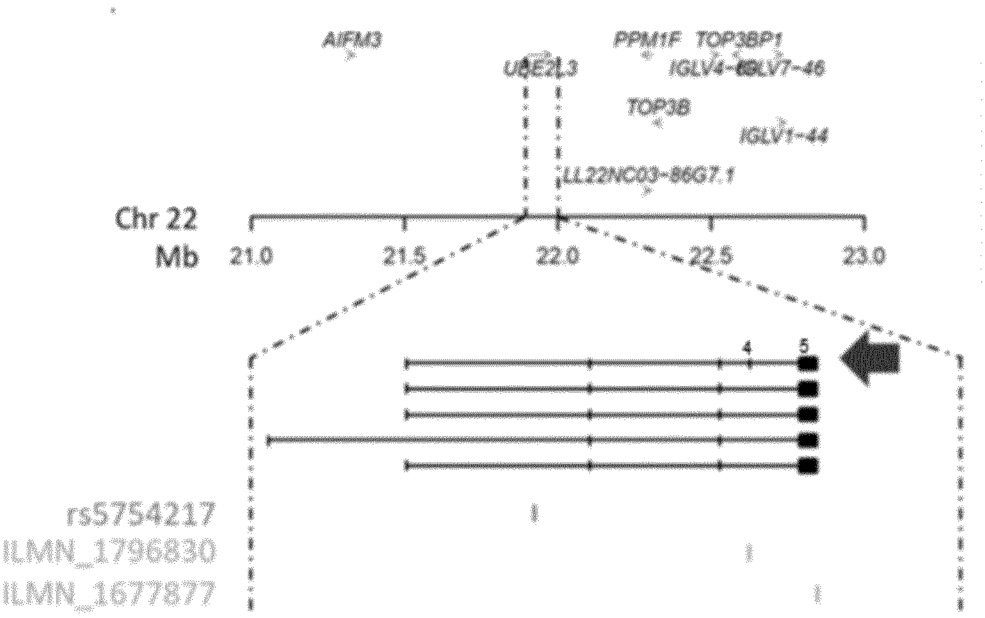
AIFM3
UBE2L3
PPM1F
TOP3BP1
IGLV4-69
IGLV7-46
TOP3B
IGLV1-44
LL22NC03-86G7.1
Chr 22
Mb
21.0
21.5
22.0
22.5
23.0
4
5
rs5754217
ILMN_1796830
ILMN_1677877

FIG. 1

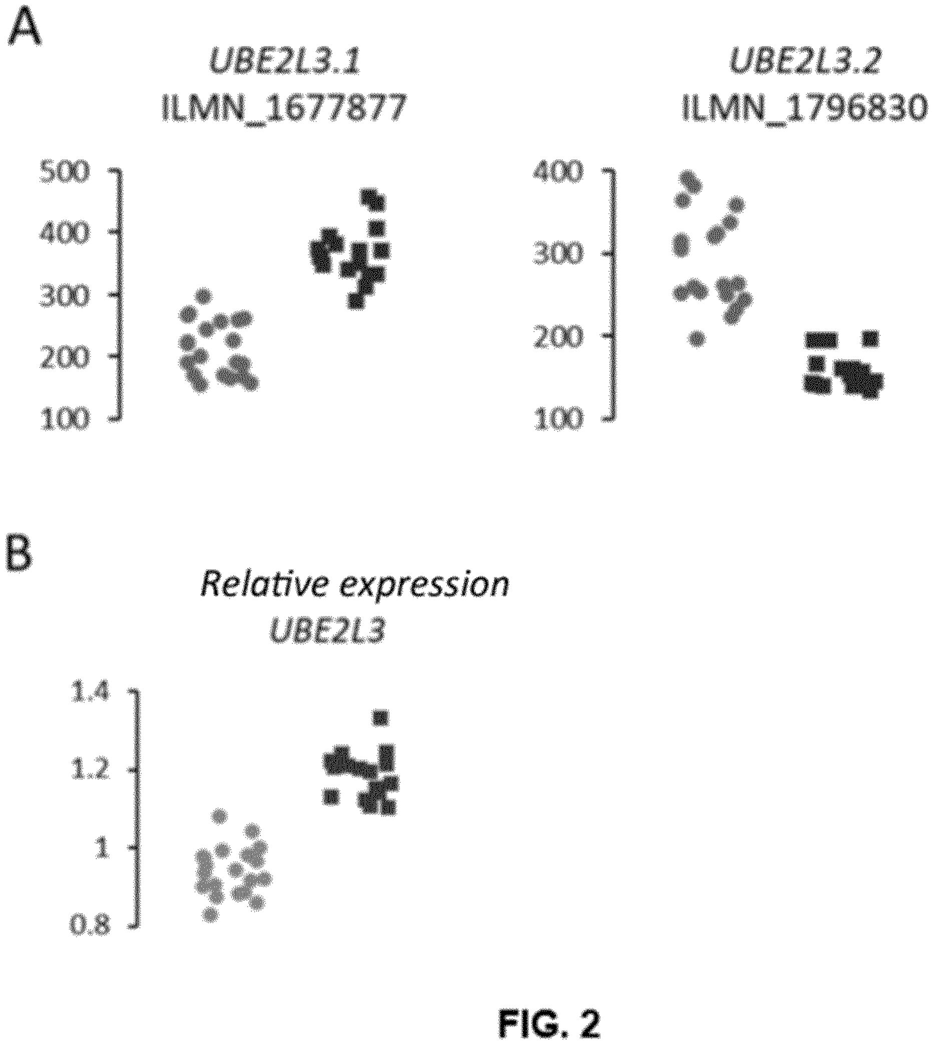
A
UBE2L3.1
ILMN_1677877
500
400
300
200
100
UBE2L3.2
ILMN_1796830
400
300
200
100
B
Relative expression
UBE2L3
1.4
1.2
1
0.8

FIG. 2

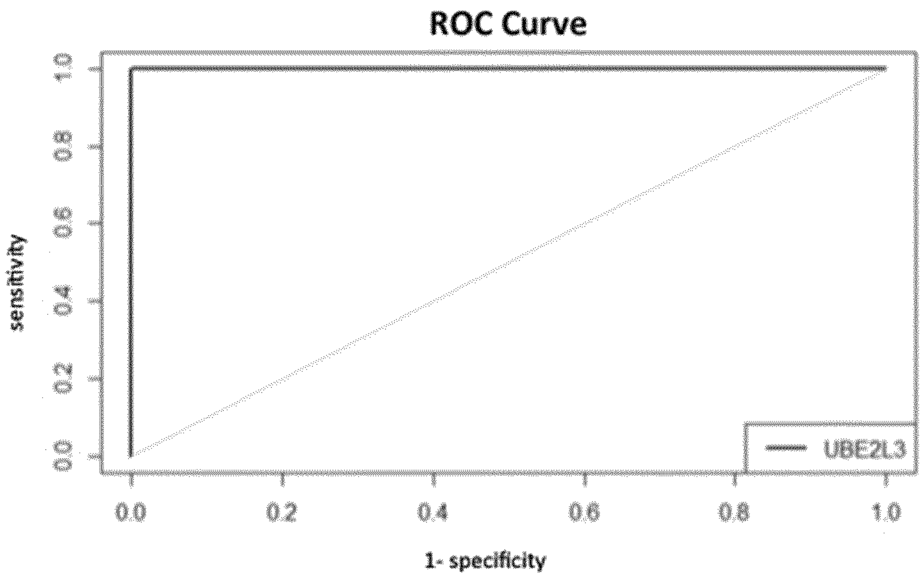
ROC Curve
sensitivity
1.0
0.8
0.6
0.4
0.2
0.0
UBE2L3
0.0
0.2
0.4
0.6
0.8
1.0
1- specificity

FIG. 3

## INFORME DE BÚSQUEDA INTERNACIONAL

Solicitud internacional Nº
PCT/ES2020/070180

A. CLASIFICACIÓN DEL OBJETO DE LA SOLICITUD
INV. C12Q1/6883
ADD.
De acuerdo con la Clasificación Internacional de Patentes (CIP) o según la clasificación nacional y CIP.

B. SECTORES COMPRENDIDOS POR LA BÚSQUEDA

Documentación mínima buscada (sistema de clasificación seguido de los símbolos de clasificación)
C12Q

Otra documentación consultada, además de la documentación mínima, en la medida en que tales documentos formen parte de los sectores comprendidos por la búsqueda

Bases de datos electrónicas consultadas durante la búsqueda internacional (nombre de la base de datos y, si es posible, términos de búsqueda utilizados)
EPO-Internal, BIOSIS, Sequence Search, EMBASE, WPI Data

C. DOCUMENTOS CONSIDERADOS RELEVANTES

| Categoría* | Documentos citados, con indicación, si procede, de las partes relevantes | Relevante para las reivindicaciones Nº |
|---|---|---|
| X | V. PASCUAL ET AL: "Different Gene Expression Signatures in Children and Adults with Celiac Disease", PLOS ONE, vol . 11, no. 2, 9 de February de 2016 (2016-02-09) , página e0146276, XP055714587, DO I : 10.1371/ journal .pone.0146276<br>Resumen; figura 1; tabla 1<br>Página 2<br>-----<br>-/-- | 1-14 |

☒ En la continuación del Recuadro C se relacionan otros documentos ☒ Los documentos de familias de patentes se indican en el Anexo

* Categorías especiales de documentos citados:
"A" documento que define el estado general de la técnica no considerado como particularmente relevante.
"E" solicitud de patente o patente anterior pero publicada en la fecha de presentación internacional o en fecha posterior.
"L" documento que puede plantear dudas sobre una reivindicación de prioridad o que se cita para determinar la fecha de publicación de otra cita o por una razón especial (como la indicada).
"O" documento que se refiere a una divulgación oral, a una utilización, a una exposición o a cualquier otro medio.
"P" documento publicado antes de la fecha de presentación internacional pero con posterioridad a la fecha de prioridad reivindicada.
"T" documento ulterior publicado con posterioridad a la fecha de presentación internacional o de prioridad que no pertenece al estado de la técnica pertinente pero que se cita por permitir la comprensión del principio o teoría que constituye la base de la invención.
"X" documento particularmente relevante; la invención reivindicada no puede considerarse nueva o que implique una actividad inventiva por referencia al documento aisladamente considerado.
"Y" documento particularmente relevante; la invención reivindicada no puede considerarse que implique una actividad inventiva cuando el documento se asocia a otro u otros documentos de la misma naturaleza, cuya combinación resulta evidente para un experto en la materia.
"&" documento que forma parte de la misma familia de patentes.

| Fecha en que se ha concluido efectivamente la búsqueda internacional. | Fecha de expedición del informe de búsqueda internacional |
|---|---|
| 16 de Julio de 2020 | 24 de Julio de 2020 |
| Nombre y dirección postal de la Administración encargada de la búsqueda internacional<br>E P O<br>P.B. 5818 Patentlaan 2<br>NL - 2280 HV Rijswijk<br>Tel. (+31-70) 340-2040,<br>Fax: (+31-70) 340-3016<br>Nº de fax | Funcionario autorizado<br>Reuter, Uwe<br>Nº de teléfono |

Formulario PCT/ISA/210 (segunda hoja) (Enero 2015)

**INFORME DE BÚSQUEDA INTERNACIONAL**

| Solicitud internacional Nº |
|---|
| PCT/ES2020/070180 |

| C (continuación). DOCUMENTOS CONSIDERADOS RELEVANTES | | |
|---|---|---|
| Categoría* | Documentos citados, con indicación, si procede, de las partes relevantes | Relevante para las reivindicaciones Nº |
| A | Gutierrez Achury ET AL: "HLA and other tales: The different perspectives of Celiac Disease",<br><br>1 de Enero de 2015 (2015-01-01), XP055714598, Recuperado de Internet:<br>URL: https : //www. rug . nl /research/portal /fi 1 es/25828797/Chapter_7.pdf<br>[recuperado el 14-07-2020]<br>tabla 3<br>----- | 1-14 |
| A | W0 2016/054038 A1 (IMMUSANT INC [US])<br>7 Abril 2016 (2016-04-07)<br>Reivindicaciones<br>----- | 1-14 |
| A | SARNA VIKAS K ET AL: "HLA-DQ-Gluten Tetramer Blood Test Accurately Identifies Patients With and Without Celiac Disease in Absence of Gluten Consumption",<br>GASTROENTEROLOGY, ELSEVIER INC, US,<br>vol . 154, no. 4,<br>14 de Noviembre de 2017 (2017-11-14), página 886,<br>XP085358496,<br>ISSN: 0016-5085, D0I:<br>10.1053/J.GASTR0.2017.11.006<br>Resumen.<br>----- | 1-14 |

Formulario PCT/ISA/210 (continuación de la segunda hoja) (Enero 2015)

**INFORME DE BÚSQUEDA INTERNACIONAL**

Información relativa a miembros de familias de patentes

Solicitud internacional Nº

PCT/ES2020/070180

| | | | | | | |
|---|---|---|---|---|---|---|
| WO 2016054038 | A1 | 07-04-2016 | AU | 2015323979 | A1 | 18-05-2017 |
| | | | CA | 2962933 | A1 | 07-04-2016 |
| | | | EP | 3201354 | A1 | 09-08-2017 |
| | | | US | 2017218453 | A1 | 03-08-2017 |
| | | | US | 2020024664 | A1 | 23-01-2020 |
| | | | WO | 2016054038 | A1 | 07-04-2016 |

Formulario PCT/ISA/210 (anexo_familia de patentes) (Enero 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **SARNA VK et al.** *Gastroenterology,* 2018, vol. 154, 886-96
- **MAY-LING J et al.** *Immunogenetics,* 2010, vol. 62, 641-651
- **SAMBROOK et al.** Molecular cloning: a Laboratory Manual. Cold Spring Harbor Laboratory Press, 2001, vol. 1-3
- **CHOMCZYNSKI et al.** *Anal. Biochem.,* 1987, vol. 162, 156
- **CHOMCZYNSKI P.** *Biotechniques,* 1993, vol. 15, 532
- **J. W. MANNHALTER et al.** *Clin. Immunol. Immunopathol.,* 1986, vol. 38, 390-397
- **M. KANEHISA.** *Nucleic Acids Res.,* 1984, vol. 12, 203
- **ZHU Z et al.** *Nat Genet.,* 2016, vol. 48, 481-7
- **DUBOIS PC et al.** *Nat Genet.,* 2010, vol. 42, 295-302
- *Nature,* 2017, vol. 550, 204-13
- **LLOYD-JONES LR et al.** *Am J Hum Genet.,* 2017, vol. 100, 228-237
- **SANGINETO M et al.** *PLoS One,* 2018, vol. 13, e0197915
- **BURCZYNSKI ME et al.** *J Mol Diagn.,* 2006, vol. 8, 51-61